# EUROPEAN PATENT SPECIFICATION

(11) **EP 1 364 671 B1**
(45) Date of publication and mention of the grant of the patent: **25.10.2006**
(21) Application number: 03253044.6
(22) Date of filing: 15.05.2003
(51) Int. Cl.: A61M 11/06, A61M 16/12

(54) **Breathing device with nebuliser**
Atemvorrichtung mit einem Vernebler
Dispositif respiratoire avec nébulisateur

(30) Priority: 23.05.2002 GB 0211906
(43) Date of publication of application: 26.11.2003
(73) Proprietor: The BOC Group plc, Windlesham Surrey GU20 6HJ (GB)
(72) Inventor: Dougill, Silvia Beatriz, London SW20 8TN (GB)
(74) Representative: Wickham, Michael

(56) References cited:
- US-A- 2 605 764
- US-A- 5 586 551
- US-A- 5 697 364
- US-A1- 2001 035 181
- US-B1- 6 318 366

## Description

The present invention relates to medical apparatus for delivering a breathable gas to a patient which also enhances the delivery of nebulised medical drug/formulations to the patient.

For the avoidance of doubt the use of the term "breathable gas" throughout this specification is intended to embrace "breathable gas mixtures" for example oxygen/helium gas mixtures.

Nebulisers are known for delivering medication directly in to the airways and lungs of a patient usually for the treatment of respiratory diseases. Commonly known nebulisers are pneumatically operated using a source of compressed air which atomises a liquid prior to delivery to a patient. One problem associated with known nebulisers is that some at least of the prescribed medication never reaches the lungs of the patient. Of the medication placed in the nebuliser sometimes two thirds remains there at the end of nebulisation. Furthermore, some of the medication released from the nebulisers may be released during expiration by the patient and be lost in to the surrounding air.

US-A-5 586 551 discloses an oxygen mask having an extended nose region to accommodate openings for both a nebuliser and a main oxygen supply. The nebuliser has a separate oxygen supply tube connected to it

US-A-2001/0035181 discloses a different apparatus for administering to a patient a nebulised liquid medicament carried in a breathable gas. The nebuliser communicates with a rebreather bag that is connected to the patient's face mask. There are no one-way valves in the apparatus. A flow of pressurised gas enters the nebuliser and is effective to convert the liquid medicament to an aerosol by means of an arrangement that utilises the Bernoulli principle. The resulting mixture of gas and entrained aerosol flows via a connecting passage into the rebreather bag. There it mixes with exhaled gas which has been collected in the bag. The resultant mixture is inhaled by the patient. In an alternative embodiment, there may be an additional gas flow in the form of a mixture of helium and oxygen that is introduced directly into the connecting passage. As a result, improved flow of aerolised liquid for better delivery of the mixture to the more restricted areas of the patient's lungs is permitted.

It is an aim of the present invention to provide an apparatus which not only delivers a breathable gas to a patient but enhances the delivery of nebulised medication to the patient.

According to the present invention, an apparatus for delivering a breathable gas together with a nebulised medicament to a patient comprises a source of gas under pressure, a nebuliser, a first line extending between the source and the nebuliser such that gas from the source when passing through the line will operate the nebuliser, an outlet from the nebuliser in communication with a mask for use by the patient, the mask being formed with one or more apertures for the flow out of the mask of exhaled gas when the patient breathes out; and a second line extending between a source of breathable gas and the mask for delivering a breathable gas to the patient, characterised in that the apparatus additionally comprises a third line extending between the source of gas under pressure and the nebuliser for supplying gas to draw the nebulised medicament into the airway and lungs of the patient.

The presence of the third line enables delivery of nebulised medication to the patient to be maintained throughout the inspiratory phase of a patient's breathing cycle.

The first line preferably extends to the base of the nebuliser. The third line preferably extends to the upper end of the nebuliser.

In one embodiment, the source of gas under pressure and the source of breathable gas is a gas cylinder containing a helium/oxygen gas mixture.

In an alternative embodiment, the source of gas under pressure is a first cylinder of air or oxygen whilst the source of breathable gas is a second cylinder containing a helium/oxygen gas mixture. Alternatively, the positions of the first and second cylinders may be reversed, i.e. the first cylinder may be of a helium/oxygen gas mixture and the second cylinder of oxygen or air.

A demand valve or a conserver may be located in the second line. Another demand valve or conserver may be located in or upstream of the first line. This offers the advantage that the nebulised medicament is released from the nebuliser only during inspiration by the patient.

Embodiments of the invention will now be described, by way of example, reference being made to the Figures of the accompanying diagrammatic drawings in which:
Figure 1 is a schematic view of an apparatus for delivering a breathable gas to a patient together with a nebulised medicament according to the present invention; and
Figure 2 is a schematic view of a modified apparatus similar to the apparatus of Figure 1.

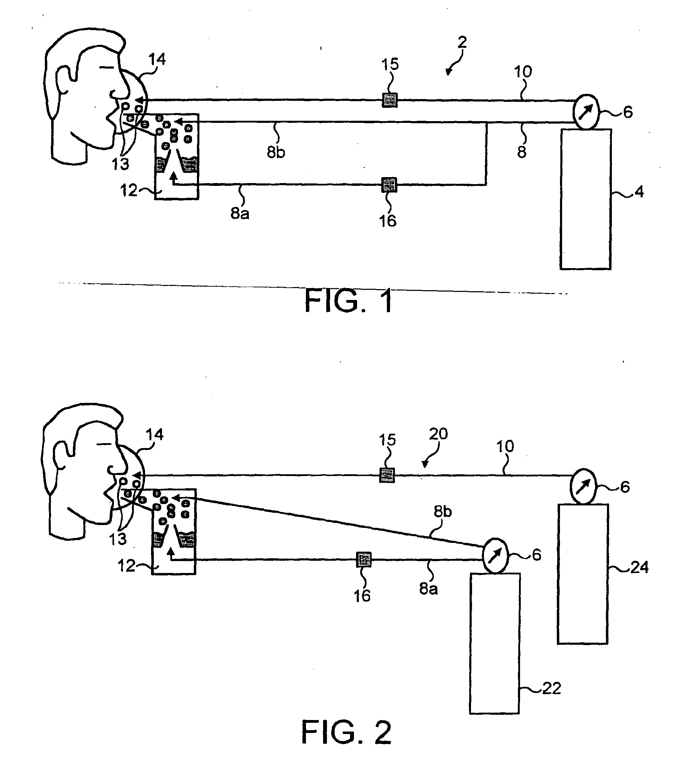

Referring first to Figure 1, there is shown an apparatus 2 for delivering a breathable gas, for example, a helium-oxygen gas mixture to a patient together with a nebulised medicament. The helium-oxygen mixture preferably contains from 20 to 40% by volume of oxygen and from 60 to 80% by volume of helium. The apparatus 2 includes a source of the gas under pressure in the form of a gas cylinder 4. A gas regulator 6 is fitted to the top (as shown) of the cylinder 4 in a manner known per se and from the regulator 6 extend first and second lines 8, 10 to a nebuliser 12 containing a liquid medicament. The outlet from the nebuliser 12 is in communication with a mask 14 for use by the patient. The mask 14 is formed with one or more apertures 13 for the flow out of the mask 14 of exhaled gas when the patient breathes out. If desired, the apertures 13 may be fitted with flap valves (not shown).

As shown, the first line 8 is branched so that branch 8a extends towards the base of the nebuliser and the branch 8b extends towards the upper end of the nebuliser. As shown, located in the branch 8a is a first demand valve 16.

The second line 10 extends directly from the regulator 6 to the mask 14 and has a second demand valve 15 disposed therein.

In operation, gas under pressure from the cylinder 4 will on inspiration by the patient, pass through line 8 and in to the nebuliser 12. The gas passing through the branch 8a will function to operate the nebuliser 12 by nebulising the liquid medicament which will then pass out from the nebuliser 12 to the mask 14 and hence in to the lungs and airways of the patient. The gas passing through the branch 8b in to the nebuliser 12 helps to draw more of the nebulised medicament in to the lungs/airways of the patient.

The gas passing continuously through the second line 10 to the mask 14 provides a constant supply of breathable gas to the patient during the inspiratory phases of his or her breathing cycle.

The demand valve 16 opens only during inspiration allowing gas to be drawn through the nebuliser 12. On expiration the valve 16 closes. If desired, the demand valve 16 can be omitted or located upstream of the branching of the line 8. Alternatively, the demand valve 16 can be replaced by a conserver.

Referring now to Figure 2 where like reference numerals denote like parts, the apparatus 20 includes a first source of gas, for example, air or oxygen under pressure in the form of a cylinder 22 and a second source of breathable gas, for example a helium/oxygen gas mixture in the form of a separate cylinder 24.

Gas regulators 6 are fitted to the respective cylinders 22, 24. A first line 8a extends between the regulator 6 of cylinder 22 and the base of the nebuliser 12 and a branch line 8b extends between the regulator 6 and the upper end of the nebuliser 12.

A second line 10 extends directly from the regulator 6 of cylinder 24 to the mask 14 for use by the patient. The mask 14 has apertures 13 in it for the exit of exhaled gas. A demand valve 15 is located in the second line 10 to ensure that gas is delivered to the patient therethrough only during the inspiratory phases of his or her breathing cycle.

As with the apparatus 2 of Figure 1, a demand valve 16 is located in the first line 8a and the outlet from the nebuliser 12 is in communication with the mask 14.

In operation, gas under pressure from the cylinder 22 will, on inspiration by the patient pass through the line 8a to operate the nebuliser 12 by nebulising liquid medicament contained within the nebuliser which will then pass out from the nebuliser to the mask 14 and hence to the lungs and airways of the patient. The gas from the cylinder 22 passing through the branch 8b in to the nebuliser 12 helps to draw more of the nebulised medicament in to the lungs/airways of the patient.

The breathable gas from the cylinder 24 passes continuously through the second line 10 to the mask 14 to provide a constant supply of breathable gas to the patient during the inspiratory phases of his or her breathing cycle.

In a modification of the apparatuses shown in Figures 1 and 2, the mask 14 may be connected to the nebuliser 12 by an adapter (not shown) having a configuration that ensures that the nebuliser is held in a vertical position. In another modification, the demand valves 15 and 16 are omitted.

Advantages of the apparatus 2, 22 over known nebulisers includes:
a) less medicament wastage;
b) improved deposition in lungs; and
c) no electrical compressors required for operating the nebuliser

## Claims

1. An apparatus for delivering a breathable gas together with a nebulised medicament to a patient comprising:
a source (4) of gas under pressure;
a nebuliser (12);
a first line (8a) extending between the source and the nebuliser such that gas from the source (4) when passing through the first line (8a) will operate the nebuliser (12);
an outlet from the nebuliser (12) in communication with a mask (14) for use by the patient, the mask (14) being formed with one or more apertures (13) for the flow out of the mask (14) of exhaled gas when the patient breathes out; and
a second line(10) extending between a source (4) of breathable gas and the mask (14) for delivering a breathable gas to the patient, **characterised in that** the apparatus additionally comprises a third line (8b) extending between the said source (4) of gas under pressure and the nebuliser (12) for supplying gas to draw the nebulised medicament into the airway and lungs of the patient

2. An apparatus as claimed in Claim 1, in which the source (4) of gas under pressure and the source (4) of breathable gas is the same gas cylinder (4).

3. An apparatus as claimed in Claim 2, in which the gas cylinder (4) contains a helium/oxygen gas mixture.

4. An apparatus as claimed in Claim 1, in which the source of gas under pressure is a cylinder of air or oxygen and the source of breathable gas is a cylinder containing a helium/oxygen gas mixture, or vice versa.

5. An apparatus as claimed in any one of Claims 1 to 4 in which a demand valve (15) is located in the second line (10).

6. An apparatus as claimed in any one of Claims 1 to 4 in which a conserver is located in the second line (10).

7. An apparatus as claimed in any one of the preceding claims, in which the first line (8a) extends to the base of the nebuliser (12).

8. An apparatus as claimed in any one of the preceding claims in which the third line (8b) extends to the upper end of the nebuliser (12).

## Patentansprüche

1. Gerät zur Abgabe eines atembaren Gases zusammen mit einem zerstäubten Medikament zu einem Patienten, mit:
einer Quelle (4) von Gas unter Druck,
einem Zerstäuber (12),
einer ersten Leitung (8a), die zwischen der Quelle und dem Zerstäuber derart verläuft, daß Gas von der Quelle (4) beim Hindurchpassieren durch die erste Leitung (8a) den Zerstäuber betreibt,
einem Auslaß aus dem Zerstäuber (12), der in Verbindung mit einer Maske (14) zum Gebrauch durch den Patienten steht, wobei die Maske (14) mit einer oder mehreren Öffnungen (13) für die Strömung eines ausgeatmeten Gases aus der Maske (14) beim Ausatmen des Patienten ausgebildet ist, und
einer zweiten Leitung (10), die zwischen einer Quelle (4) von atembarem Gas und der Maske (14) zum Zuführen eines atembaren Gases zum Patienten führt, **dadurch gekennzeichnet, daß** das Gerät zusätzlich eine dritte Leitung (8b) aufweist, die zwischen der Quelle (4) von Gas unter Druck und dem Zerstäuber (12) verläuft, um Gas zum Mitnehmen des zerstäubten Medikaments in den Luftweg und die Lunge des Patienten zuzuführen.

2. Gerät nach Anspruch 1, wobei die Quelle (4) von Gas unter Druck und die Quelle (4) von atembarem Gas durch die gleiche Gasflasche (4) gebildet sind.

3. Gerät nach Anspruch 2, wobei die Gasflasche (4) ein Helium/Sauerstoff-Gasgemisch enthält.

4. Gerät nach Anspruch 1, wobei die Quelle von Gas unter Druck eine Flasche mit Luft oder Sauerstoff und die Quelle von atembarem Gas eine Flasche mit einem Helium/Sauerstoff-Gasgemisch ist, oder umgekehrt.

5. Gerät nach einem der Ansprüche 1 bis 4, wobei ein Bedarfsventil (15) in der zweiten Leitung (10) angeordnet ist.

6. Gerät nach einem der Ansprüche 1 bis 4, wobei ein Sparventil in der zweiten Leitung (10) angeordnet ist.

7. Gerät nach einem der vorhergehenden Ansprüche, wobei die erste Leitung (8a) zur Basis des Zerstäubers (12) verläuft.

8. Gerät nach einem der vorhergehenden Ansprüche, wobei die dritte Leitung (8b) zum oberen Ende des Zerstäubers (12) verläuft.

## Revendications

1. Dispositif destiné à fournir à un patient un gaz respirable en même temps qu'un médicament nébulisé, comprenant :
une source (4) de gaz sous pression ;
un nébuliseur (12) ;
un premier conduit (8a) s'étendant entre la source et le nébuliseur de telle sorte que le gaz en provenance de la source (4), lorsqu'il passe dans le premier conduit (8a), actionne le nébuliseur (12) ;
une sortie du nébuliseur (12) en communication avec un masque (14) destiné à utilisation par le patient, le masque (14) comportant une ou plusieurs ouvertures (13) pour l'écoulement hors du masque (14) du gaz exhalé lorsque le patient exhale ; et
un deuxième conduit (10) s'étendant entre une source (4) de gaz respirable et le masque (14) pour fournir un gaz respirable au patient, ***caractérisé en ce que*** le dispositif comprend en plus un troisième conduit (8b) s'étendant entre ladite source (4) de gaz sous pression et le nébuliseur (12) pour fournir du gaz pour attirer le médicament nébulisé dans les voies aériennes et les poumons du patient.

2. Dispositif selon la revendication 1, dans lequel la source (4) de gaz sous pression et la source (4) de gaz respirable sont le même cylindre (4) de gaz.

3. Dispositif selon la revendication 2, dans lequel le cylindre de gaz (4) contient un mélange gazeux hélium/oxygène.

4. Dispositif selon la revendication 1, dans lequel la source de gaz sous pression est un cylindre d'air ou d'oxygène et la source de gaz respirable est un cylindre contenant un mélange gazeux hélium/oxygène, ou vice versa.

5. Dispositif selon l'une quelconque des revendications 1 à 4, dans lequel une soupape à la demande (ou pulmocommande) (15) est située dans le deuxième conduit (10).

6. Dispositif selon l'une quelconque des revendications 1 à 4, dans lequel un dispositif économiseur est situé dans le deuxième conduit (10).

7. Dispositif selon l'une quelconque des revendications précédentes, dans lequel le premier conduit (8a) s'étend jusqu'à la base du nébuliseur (12).

8. Dispositif selon l'une quelconque des revendications précédentes, dans lequel le troisième conduit (8b) s'étend jusqu'à l'extrémité supérieure du nébuliseur (12).
